# EUROPEAN PATENT APPLICATION

(11) **EP 2 742 969 A1**
(43) Date of publication of application: **18.06.2014**
(21) Application number: 12197001.6
(22) Date of filing: 13.12.2012
(51) Int. Cl.: A61N 1/05

(54) **A lead, especially a lead for neural applications**

(71) Applicant: Sapiens Steering Brain Stimulation B.V., 5656 AE Eindhoven (NL)
(72) Inventor: Elahi, Bijan, 5611 AZ Eindhoven (NL)
(74) Representative: Prinz & Partner

(57) **Abstract**

The present invention relates to a lead (300), especially a lead (300) for neural applications, preferably a lead (300) for a neurostimulation and/or neurorecording system (100), wherein the lead (300) comprises at least one transformable element (350), wherein the transformable element (350) is a UV-sensitive transformable element (350) which is configured such that it increases in stiffness upon exposure to UV-light. Moreover, the present invention relates to method of manufacturing and/or transforming a lead (300), further to a probe (130), a carrier (302), a protective tubing element (200) and a neurostimulation and/or neurorecording system (100).

## Description

The present invention relates to a lead, especially a lead for neural applications, preferably a lead for a neurostimulation and/or neurorecording system. Moreover, the present invention relates to a method of manufacturing and/or transforming a lead a probe, further to a carrier, a protective tubing element and a neurostimulation and/or neurorecording system.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylet material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, e.g. 1.27 mm-diameter, 10-50 cm-long probes are connected to the Implantable Pulse Generator using a 3.8 mm-diameter, 4 screw-contacts connector, by means of 110 mm-long, 2.8 mm-diameter connector cables. The proximal end of the probe has four concentric contacts that fit into the 4-contacts connector and the body of the probe is cylindrical until its end. As a consequence, the probe can pass easily through the microdrive assembly that guides the flexible probe and its stiff guiding wire into the brain tissue to the target.

Future systems will need more, smaller electrodes, in order to better control the delivery of electrical stimulation, because current stimulation causes mild to severe side-effects in about 30% of the patients. A larger number of electrodes mean a larger number of contacts to the connector, which in turn calls for different connector technologies, because it cannot be expected from the neurosurgeon to tighten 10 or more individual screws for the 10 or more contacts. Also, the contact sizes need to be smaller, certainly in the case of cranial implants.

The thin film of such a lead is fragile and can be damaged under crushing (from mechanical shocks on the head or clamping forces from the burr-plug) or sharp bending angles. Although one way to provide protection of the thin film is to increase the diameter of the lead, this option is not feasible due to increased track length and hence increased track resistance, and further incompatibility with microdrive guides.

It is desired to keep the lead diameter small-commensurate with existing leads in the market (1.27 mm OD), while simultaneously provide protection of the thin film.

It is therefore an object of the present invention, to improve a lead, a method of manufacturing and/or transferring a lead, further a probe, a carrier, a protective tubing element and a neurostimulation and/or neurorecording system, particularly in that the overall reliability and safety of the whole system may be increased and that damages of the frangible thin film of the lead of the probe may be avoided and to create protection of the lead where protection is needed without substantially increasing the diameter or length of the lead in the distal section of the lead.

The above object is solved according to the present invention by a lead according to claim 1. Accordingly, a lead is provided, especially a lead for neural applications, preferably a lead for a neurostimulation and/or neurorecording system, wherein the lead comprises at least one transformable element, wherein the transformable element is a UV-sensitive transformable element which is configured such that it increases in stiffness upon exposure to UV-light.

The lead can be e.g. a lead for neural applications or, more specifically for brain applications, preferably for a neurostimulation and/or neurorecording system. Such a neurostimulation and/or neurorecording system may be e.g. a DBS system.

By this, the overall reliability and safety of the whole system is increased and damages of the frangible thin film of the lead of the probe can be avoided. Furthermore, protection of the lead can be assured where protection is needed without substantially increasing the diameter or length of the lead in the distal section of the lead.

Moreover, the present invention allows in particular due to the use of the UV-sensitive transformable element an increase in stiffness upon exposure to UV light. For example, the at the implant time, the surgeon may implant the lead into the brain, then exposes the UV-sensitive transformable element of the lead at least partially to UV-light. For example, this part of the UV-sensitive transformable element may be the part that is outside the brain. So, the lead becomes personalized for the patient in that the part that is in the brain remains soft and flexible, but the part that is outside the brain become harder and thus more resistant to crushing forces. This is in particular of high advantage, when the lead comprises a thin film, since the thin film may be protected very efficiently according to the present invention.

For example the lead may comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or merely the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

As already outlined above, according to the present invention the advantage is achieved that the Lead becomes personalized for the patient in that the part that is in the brain remains soft and flexible, but the part that is outside the brain become harder and thus more resistant to crushing forces, which is very effective to avoid a damage of the thin film of the lead.

The transformable element may be e.g. connected and/or configured such that it may be connected to an Advanced Lead Can element. The Advanced Lead Can element may comprise electronic means to address the plurality of electrodes and at least one Advanced Lead Can connecting means. Further, the Advanced Lead Can element may be hermetically or merely hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 64 electrodes or more. The electrodes may be arranged such that the electrodes are merely evenly distributed arranged all over the distal end of the lead.

It is possible that in a further embodiment the transformable element is a carrier. Such a carrier may be a carrier for a thin film, said carrier providing the mechanical configuration of the DBS lead and the thin film. In particular, the thin film may be wound around the carrier. The carrier may be e.g. a core tube or a stylet or a bar or the like.

For instance, the material of the carrier and all further elements surrounding the carrier may be a transparent material and/or a material which is at least partially transparent and/or a material which is at least partially light-transmissive for UV-light. For example, UV-light could be induced via one end of the carrier in order to increase at least partially the stiffness/hardness of the carrier.

Moreover, it is possible that the lead is a lead comprising a thin film, wherein the thin film is at least partially wound around the carrier.

Additionally, it is possible that the carrier is configured such that the carrier is partially transformed so that it has at least a first section in which the stiffness upon exposure to UV-light is increased and at least a second section in which the stiffness upon exposure to UV-light is not increased. For example, after implantation the first section may be outside of the brain and the second section may be inside of the brain. So, the carrier of the lead and as a result the lead becomes personalized for the patient in that the part that is in the brain remains soft and flexible, but the part that is outside the brain become harder and thus more resistant to crushing forces.

Furthermore, it is possible that the transformable element is a protective tubing element which is at least partially arranged over and/or around the lead.

For example, the protective tubing element may at least partially consist of an at least partially transparent and/or light-transmissive material. In particular, it is possible that the protective tubing element at least partially consists of an UV-light-transmissive material.

Additionally, it is possible that the lead comprises a proximal end and a distal end, wherein the protective tubing element is at least partially arranged over and/or around the proximal end of the lead. The proximal end of the lead is the section of the lead which is connected or connectable to the Advanced Lead Can element. Consequently, in this section of the lead protection of the lead and the thin film is preferred and of advantage, since the proximal end of the lead may be subject to bending or the like and crushing forces.

Moreover, it is possible that the protective tubing element is configured such that the protective tubing element is partially transformed so that it has at least a first section in which the stiffness upon exposure to UV-light is increased and at least a second section in which the stiffness is not increased by lack of exposure to UV-light. For example, after implantation the first section may be outside of the brain and the second section may be inside of the brain. So, the protective tubing element of the lead and as a result the lead becomes personalized for the patient in that the part that is in the brain remains soft and flexible, but the part that is outside the brain become harder and thus more resistant to crushing forces.

Furthermore, the present invention relates to a method of manufacturing and/or transforming a lead with the features of claim 8. Accordingly, a method of manufacturing and/or transforming a lead is provided, especially a method of manufacturing and/or transforming a lead for neural applications, preferably a method of manufacturing and/or transforming a lead for a neurostimulation and/or neurorecording system, wherein the lead comprises at least one transformable element, wherein the transformable element is a UV-sensitive transformable element wherein the stiffness of the transformable element is increased upon exposure to UV-light.

In particular, it is possible that the lead is a lead according to any of claims 1 to 7.

Moreover, the present invention relates to a probe with the features of claim 10. Accordingly, a probe comprising at least one lead according to any of claims 1 to 7 is provided.

Further, the present invention relates to a carrier with the features of claim 11. Accordingly, a carrier is provided comprising the carrier features according to any of the claims 2 to 4.

Additionally, the present invention relates to a protective tubing element with the features of claim 12. Accordingly, a protective tubing element is provided comprising the protective tubing element features according to any of the claims 5 to 7.

Furthermore, the present invention relates to a neurostimulation and/or neurorecording system with the features of claim 13. Accordingly, a neurostimulation and/or neurorecording system is provided, especially a deep brain stimulation (DBS) system, comprising at least one lead according to one of claims 1 to 7 and/or at least one probe according to claim 10 and/or carrier according to claim 11 and/or protective tubing element according to claim 12.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe neurostimulation system for deep brain stimulation (DBS) and its components;
- Fig. 3:: a schematical drawing of a probe system according to the present invention;
- Fig. 4:: a perspective view distal part of the lead according to the present invention; and
- Fig. 5:: a perspective view of the protective tubing element according to the present invention.

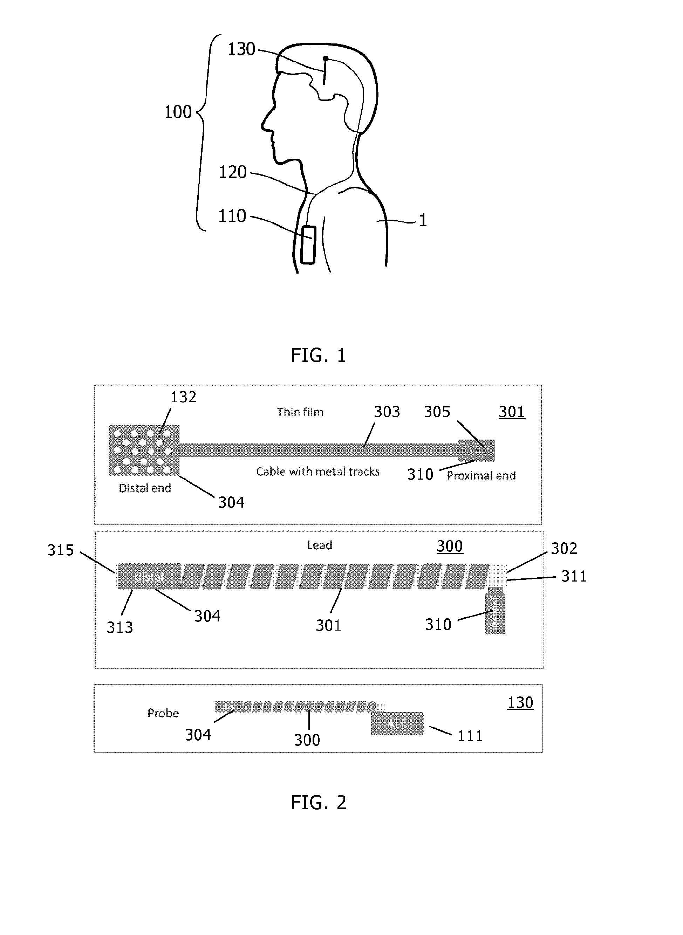

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Advanced Lead Can element 111. The Advanced Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

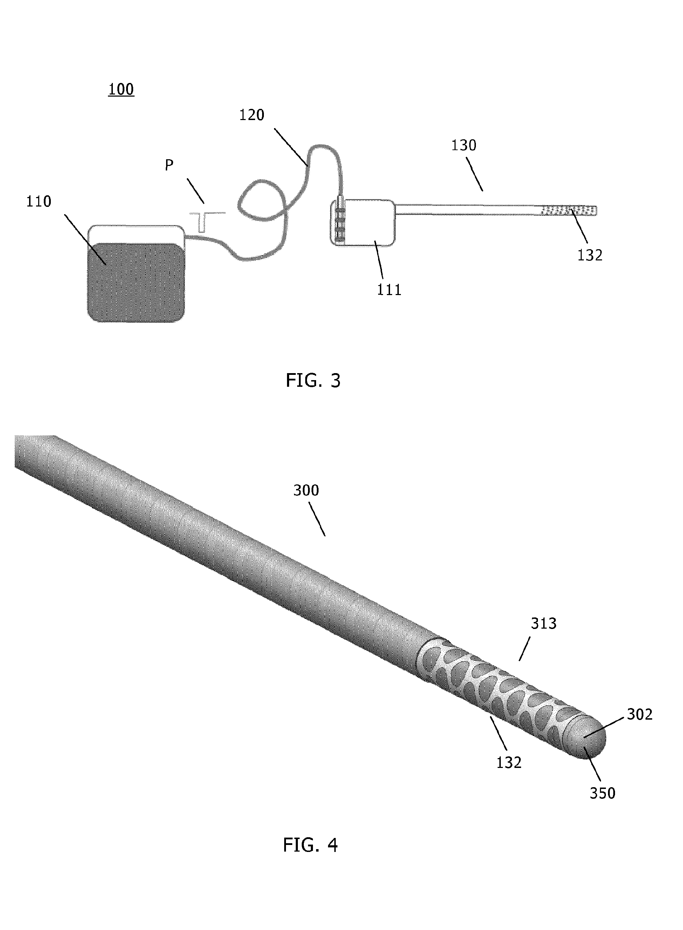

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the Advanced Lead Can 111. The controller 110 can be an implantable pulse generator (IPG) 110.

Figure 4 shows a perspective view distal part 313 of the lead 300 with a plurality of electrodes 132 according to the present invention. The lead 300 shown in Figure 4 is a lead 300 for a neurostimulation and/or neurorecording system 100 (see Figures 1-3).

The lead 300 comprises a transformable element 350, wherein the transformable element 350 is a UV-sensitive transformable element 350 which is configured such that it increases in stiffness upon exposure to UV-light.

This transformable element 350 is a carrier 302, wherein the carrier 302 for the thin film 301 as described above, said carrier 302 providing the mechanical configuration of the DBS lead 300 and the thin film 301.

Further, the carrier 302 is configured such that the carrier 302 is partially transformed so that it has at least a first section in which the stiffness upon exposure to UV-light is increased and at least a second section in which the stiffness upon exposure to UV-light is not increased. So, the carrier 302 of the lead 300 and as a result the lead 300 becomes personalized for the patient in that the part that is in the brain remains soft and flexible, but the part that is outside the brain become harder and thus more resistant to crushing forces that may e.g. damage the thin film 301.

The carrier 302 is made of a transparent and UV-light-transmissive material. This stiffness/hardness of the carrier 302 may be increased by inducing UV-light e.g. from the proximal tip end of the carrier 302 into the carrier 302. Additionally, it is possible that the surrounding elements surrounding the carrier 302 are at least partially transparent and UV-light-transmissive.

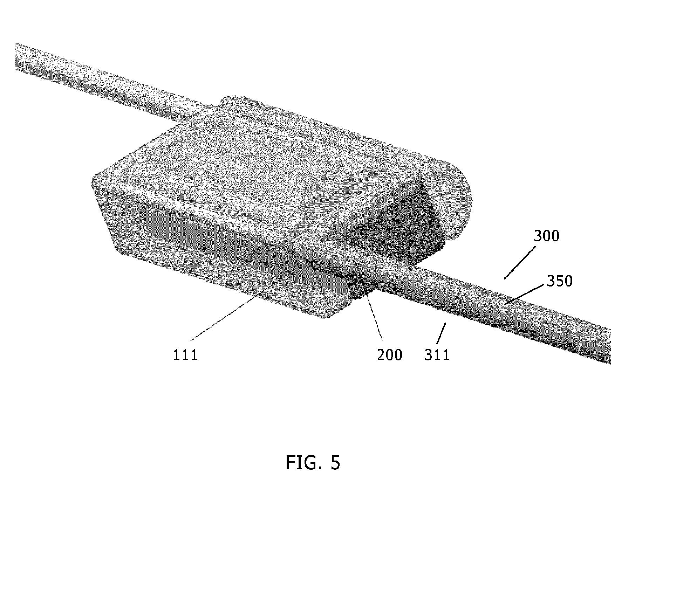

Figure 5 shows a perspective view of the protective tubing element 200 according to the present invention.

Here, the transformable element 350 is a protective tubing element 200 which is at least partially arranged over and around the lead 300 and connected to the Advanced Lead Can 111. So, the protective tubing element 200 is a UV-sensitive transformable element 350 which is configured such that it increases in stiffness upon exposure to UV-light.

The protective tubing element 200 is at least partially arranged over and around the proximal end 311 of the lead 300. The protective tubing element 200 is configured such that the protective tubing element 200 is partially transformed so that it has at least a first section in which the stiffness upon exposure to UV-light is increased and at least a second section in which the stiffness upon exposure to UV-light is not increased. So, the protective tubing element 200 of the lead 300 and as a result the lead 300 becomes personalized for the patient in that the part that is in the brain remains soft and flexible, but the part that is outside the brain become harder and thus more resistant to crushing forces that may e.g. damage the thin film 301.

The protective tubing element 200 preferably consists of a light-transmissive, in particular UV-light-transmissive and transparent material.

It is possible that a lead 300 comprises the carrier 302 according to the embodiment shown in Figure 4 and the protective tubing element 200 according to Figure 5. However, this is not mandatory to realize the present invention and its advantages, since the technical effect of the present invention may e.g. be achieved with either the embodiment of Figure 4 or 5 alone.

## Claims

1. A lead (300), especially a lead (300) for neural applications, preferably a lead (300) for a neurostimulation and/or neurorecording system (100), wherein the lead (300) comprises at least one transformable element (350), wherein the transformable element (350) is a UV-sensitive transformable element (350) which is configured such that it increases in stiffness upon exposure to UV-light.

2. The lead (300) according to claim 1,
**characterized in that**
transformable element (350) is a carrier (302).

3. The lead (300) according to claim 2,
**characterized in that**
the lead (300) is a lead (300) comprising a thin film (301), wherein the thin film is at least partially wound around the carrier (302).

4. The lead (300) according to claim 2 or 3,
**characterized in that**
the carrier (302) is configured such that the carrier (302) is partially transformed so that it has at least a first section in which the stiffness upon exposure to UV-light is increased and at least a second section in which the stiffness upon exposure to UV-light is not increased.

5. The lead (300) according to any of the preceding claims,
**characterized in that**
the transformable element (350) is a protective tubing element (200) which is at least partially arranged over and/or around the lead (300).

6. The lead (300) according to claim 5,
**characterized in that**
the lead (300) comprises a proximal end (311) and a distal end (313), wherein the protective tubing element (200) is at least partially arranged over and/or around the proximal end (311) of the lead (300).

7. The lead (300) according to claim 5 or 6,
**characterized in that**
the protective tubing element (200) is configured such that the protective tubing element (200) is partially transformed so that it has at least a first section in which the stiffness upon exposure to UV-light is increased and at least a second section in which the stiffness upon exposure to UV-light is not increased.

8. A method of manufacturing and/or transforming a lead (300), especially a lead (300) for neural applications, preferably a lead (300) for a neurostimulation and/or neurorecording system (100), wherein the lead (300) comprises at least one transformable element (350), wherein the transformable element (350) is a UV-sensitive transformable element (350) wherein the stiffness of the transformable element (350) is increased upon exposure to UV-light.

9. The method of manufacturing and/or transforming a lead (300) according to claim 8,
**characterized in that**
the lead (300) is a lead (300) according to any of claims 1 to 7.

10. A probe (130) comprising at least one lead (300) according to any of claims 1 to 7.

11. A carrier (302) comprising the carrier features according to any of the claims 2 to 4.

12. A protective tubing element (200) comprising the protective tubing element features according to any of the claims 5 to 7.

13. A neurostimulation and/or neurorecording system (100), especially a deep brain stimulation (DBS) system (100), comprising at least one lead (300) according to one of claims 1 to 7 and/or at least one probe (130) according to claim 10 and/or carrier (302) according to claim 11 and/or protective tubing element (200) according to claim 12.
